# EUROPEAN PATENT SPECIFICATION

(11) **EP 2 534 491 B1**
(45) Date of publication and mention of the grant of the patent: **19.10.2016**
(21) Application number: 11742790.6
(22) Date of filing: 10.02.2011
(51) Int. Cl.: G01N 33/573, G01N 33/68, G01N 33/53

(54) **COMPOSITONS AND METHODS FOR PREDICTING CARDIOVASCULAR EVENTS**
ZUSAMMENSETZUNGEN UND VERFAHREN ZUR VORHERSAGE KARDIOVASKULÄRER EREIGNISSE
COMPOSITIONS ET MÉTHODES POUR LA PRÉDICTION D'ÉVÉNEMENTS CARDIOVASCULAIRES

(30) Priority: 10.02.2010 US 303126 P
(43) Date of publication of application: 19.12.2012
(73) Proprietor: Cleveland Heartlab LLC, Cleveland, Ohio 44106 (US)
(72) Inventor: ORVILLE, Jacob A., Shaker Heights, Ohio 44122 (US); PURVIS, Scott, Olmsted Falls, Ohio 44138 (US); BEIDELSCHIES, Michelle, Medina, Ohio 44256 (US)
(74) Representative: Glawe, Delfs, Moll
(86) International application number: PCT/US2011/024345
(87) International publication number: WO 2011/100426

(56) References cited:
- WO-A1-2009/132510
- WO-A2-02/062207
- CA-A1- 2 796 391
- US-A1- 2006 099 608
- US-A1- 2006 105 419
- US-A1- 2006 286 681
- US-A1- 2008 213 746
- US-A1- 2009 274 709
- US-B1- 7 459 286
- RANA J S ET AL: "Inflammatory biomarkers and the prediction of coronary events among people at intermediate risk: the EPIC-Norfolk prospective population study", HEART, BRITISH CARDIAC SOCIETY, UK, vol. 95, no. 20, 1 October 2009 (2009-10-01), pages 1682-1687, XP009170516, ISSN: 1468-201X, DOI: 10.1136/HRT.2009.170134 [retrieved on 2009-07-08]
- TSIMIKAS S ET AL: "C-Reactive Protein and Other Emerging Blood Biomarkers to Optimize Risk Stratification of Vulnerable Patients", JOURNAL OF THE AMERICAN COLLEGE OF CARDIOLOGY, ELSEVIER, NEW YORK, NY, US, vol. 47, no. 8, 18 April 2006 (2006-04-18) , pages C19-C31, XP028006134, ISSN: 0735-1097, DOI: 10.1016/J.JACC.2005.10.066 [retrieved on 2006-04-18]
- KUO-CHIEN TSAO ET AL: "Multiple risk markers for atherogenesis associated with chronic inflammation are detectable in patients with renal stones", JOURNAL OF CLINICAL LABORATORY ANALYSIS, vol. 21, no. 6, 1 January 2007 (2007-01-01), pages 426-431, XP55067667, ISSN: 0887-8013, DOI: 10.1002/jcla.20215
- MARTIN-VENTURA J L ET AL: "Biomarkers in Cardiovascular Medicine", REVISTA ESPANOLA DE CARDIOLOGIA, EDICIONES DOYMA S.A., BARCELONA, ES, vol. 62, no. 6, 1 June 2009 (2009-06-01), pages 677-688, XP026644185, ISSN: 1885-5857, DOI: 10.1016/S1885-5857(09)72232-7 [retrieved on 2009-06-01]

## Description

### FIELD OF THE INVENTION

The present invention is set out in the claims and relates to methods for determining the risk of peri-operative cardiovascular events or predicting cardiovascular events associated with angioplasty. In certain embodiments, the present invention provides methods, for determining the value of five different markers selected from myeloperoxidase (MPO), an F2-isoprostane (F2-IsoP), C-reactive protein (CRP), urinary micro-albumin (UMA), and lipoprotein-associated phospholipase A2 (Lp-PLAZ), such that a subject's risk of experiencing a cardiovascular event is determined.

### BACKGROUND OF THE INVENTION

Previous efforts to risk stratify based on family history, EKG, CK-MB, troponin I testing, medical history, drugs, age, etc. have not been sufficient. There are still cardiac events occurring post- and intra-operative. Moreover, the current testing is diagnostic, not prognostic to provide more risk stratification. Tsimirkas et al., Journal of the American College of Cardiology, Vol. 47, No. 8 Suppl C, (2006), pages C19-C31 discuss C-reactive protein and other emerging blood biomarkers in the context of cardiovascular disease. Kuo-Chien Tsao et al. study the detection of risk markers for atherogenesis associated with chronic inflammation in patients with renal stones.

### SUMMARY OF THE INVENTION

The present invention is set out in the appended claims. The embodiments of the description which do not fall within the scope of said claims are provided for illustrative purposes only. Provided are methods for determining the risk of peri-operative cardiovascular events or predicting cardiovascular events associated with angioplasty. In certain embodiments, the present invention provides methods comprising determining the value of five different markers selected from myeloperoxidase (MPO), an F2-isoprostane (F2-IsoP), C-reactive protein (CRP), urinary micro-albumin (UMA), and lipoprotein-associated phospholipase A2 (Lp-PLAZ), such that a subject's risk of experiencing a cardiovascular event is determined.

In particular, the present invention provides methods of determining the risk of a cardiovascular event in a subject (e.g., human subject) comprising: a) determining the value (e.g., amount of protein, amount of mRNA, activity of protein, etc.) of first, second, third, fourth, and fifth markers in a biological sample from the subject, wherein the first, second, third, fourth, and fifth markers are different and are selected from the group consisting of: myeloperoxidase (MPO), an F2-isoprostane (F2-IsoP), C-reactive protein (CRP), urinary micro-albumin (UMA); and lipoprotein-associated phospholipase A2 (Lp-PLAZ), and b) employing the values for the first, second, third, fourth and fifth markers to determine the subject's risk of experiencing a cardiovascular event.

In certain embodiments, employing the values for the first, second, third, fourth and fifth markers to determine the subject's risk of experiencing a cardiovascular event comprises comparing the value of the first marker to a first threshold value, comparing the value of the second marker to a second threshold value, comparing the value of the third marker to a third threshold value, comparing the value of the fourth marker to a fourth threshold value, and comparing the value of the fifth marker to a fifth threshold value. In other embodiments, the cardiovascular event is a peri-operative cardiovascular or a cardiovascular event associated with angioplasty. In further embodiments, the cardiovascular event is one or more of the following: non-fatal myocardial infarction, stroke, angina pectoris, transient ischemic attacks, congestive heart failure, aortic aneurysm, aortic dissection, and death. In additional embodiments, the F2-isoprostane is selected from 5-F2-IsoP, 8-F2-IsoP, 12-F2-IsoP, and 15-F2-IsoP.

In particular embodiments, the first marker comprises MPO.

In particular embodiments, the present description provides kits (or devices or compositions) for determining the risk of a cardiovascular vent in a subject comprising:
a) first reagents (e.g., in a container, such as a vial, a multiwell plate, etc.) configured to determine the value of a first marker in a biological sample from the subject, wherein the first marker is selected from the group consisting of: myeloperoxidase (MPO), an F2-isoprostane (F2-IsoP), C-reactive protein (CRP), urinary micro-albumin (UMA); and lipoprotein-associated phospholipase A2 (Lp-PLAZ), and b) second reagents (e.g., in a container, such as a vial) configured to determine the value of a second marker in the biological sample, wherein the second marker is different from the first marker and is selected from the group consisting of: MPO, F2-IsoP, CRP, UMA, and Lp-PLAZ.

In some embodiments, the first reagents are configured for detecting MPO. In other embodiments, the first reagents comprise hydrogen peroxide and a peroxidase substrate (e.g., the biological sample is plasma or serum). In additional embodiments, the first and/or second reagents comprise antibodies or an antigen-binding portion thereof. In other embodiments, the F2-isoprostane is selected from 5-F2-IsoP, 8-F2-IsoP, 12-F2-IsoP, and 15-F2-IsoP.

In certain embodiments, the kits (or devices, or compositions) further comprise c) third reagents configured to determine the value of a third marker in the biological sample, wherein the third marker is different from the first and second markers and is selected from the group consisting of: MPO, F2-IsoP, CRP, UMA, and Lp-PLAZ. In additional embodiments, the kits or devices further comprise d) fourth reagents configured to determine the value of a fourth marker in the biological sample, wherein the fourth marker is different from the first, second, and third markers and is selected from the group consisting of: MPO, F2-IsoP, CRP, UMA, and Lp-PLAZ. In further embodiments, the kits, devices, or compositions further comprise e) fifth reagents configured to determine the value of a fifth marker in the biological sample, wherein the fifth marker is different from the first, second, third, and fourth markers and is selected from the group consisting of: MPO, F2-IsoP, CRP, UMA, and Lp-PLAZ.

In other embodiments, the present description provides sample devices configured to determining the risk of a cardiovascular vent in a subject comprising: a) first reagents configured to determine the value of a first marker in a biological sample from the subject, wherein the first marker is selected from the group consisting of: myeloperoxidase (MPO), an F2-isoprostane (F2-IsoP), C-reactive protein (CRP), urinary micro-albumin (UMA); and lipoprotein-associated phospholipase A2 (Lp-PLAZ), b) second reagents configured to determine the value of a second marker in the biological sample, wherein the second marker is different from the first marker and is selected from the group consisting of: MPO, F2-IsoP, CRP, UMA, and Lp-PLAZ; and c) a solid support for holding the first and second reagents (e.g., microfluidic card, ELISA plate, or other device). In some embodiments, the sample device is configured to be placed inside a detection device (e.g., a reader device for detecting the markers and the levels of any markers present, such as a fluorescent reader).

### DESCRIPTION OF THE INVENTION

The present invention is set out in the appended claims. The embodiments of the description which do not fall within the scope of said claims are provided for illustrative purposes only. Provided are methods for determining the risk of peri-operative cardiovascular events or predicting cardiovascular events associated with angioplasty. In certain embodiments, the present invention provides methods comprising determining the value of five different markers selected from myeloperoxidase (MPO), an F2-isoprostane (F2-IsoP), C-reactive protein (CRP), urinary micro-albumin (UMA), and lipoprotein-associated phospholipase A2 (Lp-PLAZ), such that a subject's risk of experiencing a cardiovascular event is determined.

Accordingly, in some embodiments, the present invention provides methods for predicting cardiovascular events. In some embodiments, the present invention provides a panel of markers specifically linked to inflammation making it easier to predict acute risk of events and better risk stratification prior to surgery. Thus, in some embodiments, a treatment course of action can be determined based on an individual's risk assessment. For example, in some embodiments, surgery can be delayed or altered in order to implement therapeutic and diagnostic initiatives prior to and/or during surgery. Inflammatory biomarkers are not currently being used in pre-op setting. Thus, the present invention provides the advantage of being able to individualize treatment to a patient's risk profile and thus reduce cardiovascular events associated with angioplasty or other surgery (e.g., cardiac surgery).

For example, in some embodiments, it is contemplated that increasing levels of one or more of F2-isoprostanes, hs-CRP, urinary microalbumin, Lp-PLA2, and myeloperoxidase are associated with increased risk of cardiovascular events.

The isoprostanes are prostaglandin-like compounds formed in vivo from the free radical-catalyzed peroxidation of essential fatty acids (primarily arachidonic acid) without the direct action of cyclooxygenase (COX) enzyme (Morrow JD, Roberts LJ (1996). "The isoprostanes. Current knowledge and directions for future research". Biochem. Pharmacol. 51 (1): 1-9); Milne, Biomarkers. 2005 Nov;10 Suppl 1:S10-23; herein incorporated by reference). These nonclassical eicosanoids possess potent biological activity as inflammatory mediators that augment the perception of pain (Evans AR, Junger H, Southall MD, et al. (2000). "Isoprostanes, novel eicosanoids that produce nociception and sensitize rat sensory neurons". J. Pharmacol. Exp. Ther. 293 (3): 912-20; herein incorporated by reference). These compounds are accurate markers of lipid peroxidation in both animal and human models of oxidative stress. Examples of F2-isoprostanes include, but are not limited to 5-F2-IsoP, 8-F2-IsoP, 12-F2-IsoP, and 15-F2-IsoP, which are shown below:

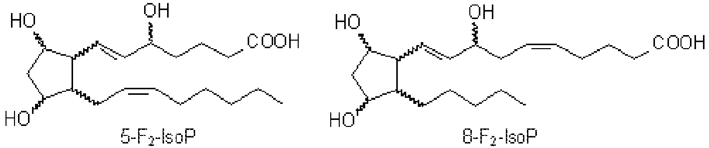

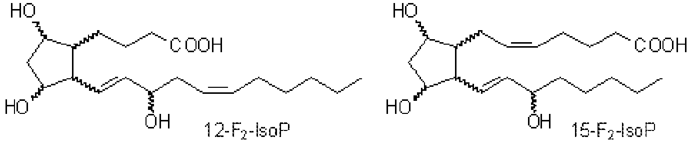

C-reactive protein (CRP) is a protein found in the blood, the levels of which rise in response to inflammation (an acute-phase protein). Its physiological role is to bind to phosphocholine expressed on the surface of dead or dying cells (and some types of bacteria) in order to activate the complement system via c1q (Thompson D, Pepys MB, Wood SP. (1999). "The physiological structure of human C-reactive protein and its complex with phosphocholine." Structure 7 (2): 169-77; herein incorporated by reference).

Urinary microalbumin is a measure of albumin in urine (James T. Wu and Lily L. Wu, Annals of Clinical & Laboratory Science 35:240-250 (2005); herein incorporated by reference).

Lipoprotein-associated phospholipase A2 (lp-PLA2) is a phospholipase A2 enzyme. It is an enzyme produced by inflammatory cells and hydrolyzes oxidized phospholipids in LDL. It is involved in the development of atherosclerosis (Zalewski, Arteriosclerosis, Thrombosis, and Vascular Biology. 2005;25:923-931; herein incorporated by reference).

Myeloperoxidase (MPO) is a peroxidase enzyme most abundantly present in neutrophil granulocytes (a subtype of white blood cells). It is a lysosomal protein stored in azurophilic granules of the neutrophil. MPO has a heme pigment, which causes its green color in secretions rich in neutrophils, such as pus and some forms of mucus (Brennan M-L, Penn MS, Van Lente F, Nambi V, Shishehbor MH, Aviles RJ, Goormastic M, Pepoy ML, McErlean ES, Topol EJ, Nissen SE, Hazen SL. Prognostic Arteriosclerosis, Thrombosis, and Vascular Biology. 2005;25:923-931; herein incorporated by reference).

Myeloperoxidase (MPO) is a peroxidase enzyme most abundantly present in neutrophil granulocytes (a subtype of white blood cells). It is a lysosomal protein stored in azurophilic granules of the neutrophil. MPO has a heme pigment, which causes its green color in secretions rich in neutrophils, such as pus and some forms of mucus (Brennan M-L, Penn MS, Van Lente F, Nambi V, Shishehbor MH, Aviles RJ, Goormastic M, Pepoy ML, McErlean ES, Topol EJ, Nissen SE, Hazen SL. Prognostic value of myeloperoxidase in patients with chest pain. N Engl J Med 2003;349:1595-604; all of which are herein incorporated by reference).

In certain embodiments, the present invention provides methods for detecting all five markers selected from the group consisting of: Myeloperoxidase (MPO); an F2-isoprostane (F2-IsoP); C-reactive protein (CRP); Urinary micro-albumin (UMA); and Lipoprotein-associated phospholipase A2 (Lp-PLAZ) in order to determine the risk of a cardiovascular event, such as determining the risk of a peri-operative cardiovascular event or predicting a cardiovascular event associated with angioplasty. In some embodiments, the F2-isoprostane is selected from 5-F2-IsoP, 8-F2-IsoP, 12-F2-IsoP, and 15-F2-IsoP.

Table 1 below provides the five exemplary markers that can be tested for in a sample, such as blood sample, plasma sample, urine sample, etc., with an analyzer (e.g., hematology analyzer; ELISA test, etc.) in order to at least partially characterize a subject's risk of cardiovascular disease or experiencing a complication of cardiovascular disease. The combinations of makers in Table 1 may be employed in a group consisting or comprising the recited markers. Table 1 is presented below.

**TABLE 1**

| **Combinations of 2 Markers** | **Combinations of 3 Markers** | **Combinations of 4 Markers** | **All 5 Markers** |
|---|---|---|---|
| 1 | 1 | 1 | 1 |
| MPO+F2-IsoP | MPO + F2-IsoP + CRP | MPO + F2-IsoP + CRP + UMA | MPO + F2-IsoP + CRP + |
| 2 | 2 | 2 | |
| F2-IsoP + Lp-PLAZ | F2-IsoP + CRP + UMA | | |
| 8 | 8 | | |
| CRP + UMA | F2-IsoP + CRP + Lp-PLAZ | | |
| 9 | 9 | | |
| CRP + Lp-PLAZ | F2-IsoP + UMA + Lp-PLAZ | | |
| 10 | 10 | | |
| UMA + Lp-PLAZ | CRP + UMA + Lp-PLAZ | | |

| | | | |
|---|---|---|---|
| Myeloperoxidase = MPO; F2-isoprostane = F2-IsoP; C-reactive protein = CRP; Urinary micro-albumin= UMA; and Lipoprotein-associated phospholipase A2= Lp-PLAZ. | | | |

It is noted that the present invention is not limited to combinations of markers comprising or consisting of five markers. Instead, any and all combinations of markers from Table 1 may be made which include, for example, groups (comprising or consisting of) six markers, seven markers, eight markers, nine markers, ten markers ... fifteen markers ... twenty markers ... thirty markers ... fifty markers ... and seventy five markers - selected from other markers that are known or used in the art to predict the risk of cardiovascular events or disease (e.g., in the peri-operative context or angioplasty context).

In some embodiments, markers are detected in urine or serum, although the invention is not limited to the nature of the sample used. Any patient sample may be tested according to the methods of the present invention. By way of non-limiting examples, the sample may be tissue, blood, urine, semen, or a fraction thereof (*e.g*., plasma, serum, urine supernatant, urine cell pellet, etc.). In some embodiments, the patient sample is processed prior to assaying for the marker to isolate or enrich the sample for the markers described herein. A variety of techniques known to those of ordinary skill in the art may be used for this purpose, including but not limited: centrifugation; immunocapture; cell lysis; and, nucleic acid target capture.

In some embodiments, markers are detected in a multiplex or panel format. In some embodiments, the assays detect quantitative levels of marker polypeptide. In some embodiments, panels, assay kits and/or methods comprise reagents and methods for detecting a panel of markers comprising or consisting of two or more of F2-isoprostanes, hs-CRP, urinary microalbumin, Lp-PLA2, and myeloperoxidase.

In some embodiments, assays are conducted before during, or after a medical procedure (e.g., surgery, drug regimen, etc.). In some embodiments, result of the assay is used to select appropriate intervention, modify intervention (e.g., drug selection, dose, timing, addition or omission of additional diagnostic or other Medical test or procedures, etc.), and test/assess the intervention.

Any suitable detection assay may be utilized in the methods described herein. Illustrative non-limiting examples of protein detection methods include protein sequencing/analysis and immunoassays.

Protein sequencing techniques/analysis include, but are not limited to, mass spectrometry and Edman degradation.

Illustrative non-limiting examples of immunoassays include, but are not limited to: immunoprecipitation; Western blot; ELISA; immunohistochemistry; immunocytochemistry; flow cytometry; and, immuno-PCR. Polyclonal or monoclonal antibodies detectably labeled using various techniques known to those of ordinary skill in the art (*e.g*., colorimetric, fluorescent, chemiluminescent or radioactive) are suitable for use in the immunoassays.

Immunoprecipitation is the technique of precipitating an antigen out of solution using an antibody specific to that antigen. The process can be used to identify protein complexes present in cell extracts by targeting a protein believed to be in the complex. The complexes are brought out of solution by insoluble antibody-binding proteins isolated initially from bacteria, such as Protein A and Protein G. The antibodies can also be coupled to sepharose beads that can easily be isolated out of solution. After washing, the precipitate can be analyzed using mass spectrometry, Western blotting, or any number of other methods for identifying constituents in the complex.

A Western blot, or immunoblot, is a method to detect protein in a given sample of tissue homogenate or extract. It uses gel electrophoresis to separate denatured proteins by mass. The proteins are then transferred out of the gel and onto a membrane, typically polyvinyldiflroride or nitrocellulose, where they are probed using antibodies specific to the protein of interest. As a result, researchers can examine the amount of protein in a given sample and compare levels between several groups.

An ELISA, short for Enzyme-Linked ImmunoSorbent Assay, is a biochemical technique to detect the presence of an antibody or an antigen in a sample. It utilizes a minimum of two antibodies, one of which is specific to the antigen and the other of which is coupled to an enzyme. The second antibody will cause a chromogenic or fluorogenic substrate to produce a signal. Variations of ELISA include sandwich ELISA, competitive ELISA, and ELISPOT. Because the ELISA can be performed to evaluate either the presence of antigen or the presence of antibody in a sample, it is a useful tool both for determining serum antibody concentrations and also for detecting the presence of antigen.

Immunohistochemistry and immunocytochemistry refer to the process of localizing proteins in a tissue section or cell, respectively, via the principle of antigens in tissue or cells binding to their respective antibodies. Visualization is enabled by tagging the antibody with color producing or fluorescent tags. Typical examples of color tags include, but are not limited to, horseradish peroxidase and alkaline phosphatase. Typical examples of fluorophore tags include, but are not limited to, fluorescein isothiocyanate (FITC) or phycoerythrin (PE).

Flow cytometry is a technique for counting, examining and sorting microscopic particles suspended in a stream of fluid. It allows simultaneous multiparametric analysis of the physical and/or chemical characteristics of single cells flowing through an optical/electronic detection apparatus. A beam of light (*e.g*., a laser) of a single frequency or color is directed onto a hydrodynamically focused stream of fluid. A number of detectors are aimed at the point where the stream passes through the light beam; one in line with the light beam (Forward Scatter or FSC) and several perpendicular to it (Side Scatter (SSC) and one or more fluorescent detectors). Each suspended particle passing through the beam scatters the light in some way, and fluorescent chemicals in the particle may be excited into emitting light at a lower frequency than the light source. The combination of scattered and fluorescent light is picked up by the detectors, and by analyzing fluctuations in brightness at each detector, one for each fluorescent emission peak, it is possible to deduce various facts about the physical and chemical structure of each individual particle. FSC correlates with the cell volume and SSC correlates with the density or inner complexity of the particle (*e.g*., shape of the nucleus, the amount and type of cytoplasmic granules or the membrane roughness).

Immuno-polymerase chain reaction (IPCR) utilizes nucleic acid amplification techniques to increase signal generation in antibody-based immunoassays. Because no protein equivalence of PCR exists, that is, proteins cannot be replicated in the same manner that nucleic acid is replicated during PCR, the only way to increase detection sensitivity is by signal amplification. The target proteins are bound to antibodies which are directly or indirectly conjugated to oligonucleotides. Unbound antibodies are washed away and the remaining bound antibodies have their oligonucleotides amplified. Protein detection occurs via detection of amplified oligonucleotides using standard nucleic acid detection methods, including real-time methods.

In some embodiments, nucleic acid expression is analyzed using any of a variety of known techniques.

In certain embodiments, the markers of the present invention are detected by hematology analyzers (e.g., MPO is detected by a hematology analyzer). A hematology analyzer (a.k.a. haematology analyzer, hematology analyzer, haematology analyser) is an automated instrument (e.g. clinical instrument and/or laboratory instrument) which analyzes the various components (e.g. blood cells) of a blood sample. Typically, hematology analyzers are automated cell counters used to perform cell counting and separation tasks including: differentiation of individual blood cells, counting blood cells, separating blood cells in a sample based on cell-type, quantifying one or more specific types of blood cells, and/or quantifying the size of the blood cells in a sample. In some embodiments, hematology analyzers are automated coagulometers which measure the ability of blood to clot (e.g. partial thromboplastin times, prothrombin times, lupus anticoagulant screens, D dimer assays, factor assays, etc.), or automatic erythrocyte sedimentation rate (ESR) analyzers. In general, a hematology analyzer performing cell counting functions samples the blood, and quantifies, classifies, and describes cell populations using both electrical and optical techniques. A properly outfitted hematology analyzer (e.g. with peroxidase staining capability) is capable of providing values for Markers 1-55, using various analyses.

Electrical analysis by a hematology analyzer generally involves passing a dilute solution of a blood sample through an aperture across which an electrical current is flowing. The passage of cells through the current changes the impedance between the terminals (the Coulter principle). A lytic reagent is added to the blood solution to selectively lyse red blood cells (RBCs), leaving only white blood cells (WBCs), and platelets intact. Then the solution is passed through a second detector. This allows the counts of RBCs, WBCs, and platelets to be obtained. The platelet count is easily separated from the WBC count by the smaller impedance spikes they produce in the detector due to their lower cell volumes.

In some embodiments, a computer-based analysis program is used to translate the raw data generated by the detection assay (*e.g*., the presence, absence, or amount of a given marker) into data of predictive value for a clinician. The clinician can access the predictive data using any suitable means. Thus, in some preferred embodiments, the present invention provides the further benefit that the clinician, who is not likely to be trained in genetics or molecular biology, need not understand the raw data. The data is presented directly to the clinician in its most useful form. The clinician is then able to immediately utilize the information in order to optimize the care of the subject.

The present invention contemplates any method capable of receiving, processing, and transmitting the information to and from laboratories conducting the assays, information provides, medical personal, and subjects. For example, in some embodiments of the present invention, a sample (*e.g*., a biopsy or a serum or urine sample) is obtained from a subject and submitted to a profiling service (*e.g*., clinical lab at a medical facility, genomic profiling business, etc.), located in any part of the world (*e.g*., in a country different than the country where the subject resides or where the information is ultimately used) to generate raw data. Where the sample comprises a tissue or other biological sample, the subject may visit a medical center to have the sample obtained and sent to the profiling center, or subjects may collect the sample themselves (*e.g*., a urine sample) and directly send it to a profiling center. Where the sample comprises previously determined biological information, the information may be directly sent to the profiling service by the subject (*e.g*., an information card containing the information may be scanned by a computer and the data transmitted to a computer of the profiling center using an electronic communication systems). Once received by the profiling service, the sample is processed and a profile is produced (*i.e*., expression data), specific for the diagnostic or prognostic information desired for the subject.

The profile data is then prepared in a format suitable for interpretation by a treating clinician. For example, rather than providing raw expression data, the prepared format may represent a diagnosis or risk assessment (*e.g*., risk of cardiovascular events) for the subject, along with recommendations for particular treatment options. The data may be displayed to the clinician by any suitable method. For example, in some embodiments, the profiling service generates a report that can be printed for the clinician (*e.g.,* at the point of care) or displayed to the clinician on a computer monitor.

In some embodiments, the information is first analyzed at the point of care or at a regional facility. The raw data is then sent to a central processing facility for further analysis and/or to convert the raw data to information useful for a clinician or patient. The central processing facility provides the advantage of privacy (all data is stored in a central facility with uniform security protocols), speed, and uniformity of data analysis. The central processing facility can then control the fate of the data following treatment of the subject. For example, using an electronic communication system, the central facility can provide data to the clinician, the subject, or researchers.

In some embodiments, the subject is able to directly access the data using the electronic communication system. The subject may chose further intervention or counseling based on the results. In some embodiments, the data is used for research use. For example, the data may be used to further optimize the inclusion or elimination of markers as useful indicators of a particular condition or stage of disease.

In certain embodiments, values of the markers of the present invention in the biological sample obtained from the test subject may compared to a threshold value. A threshold value is a concentration or number of an analyte (e.g., particular cells type) that represents a known or representative amount of an analyte. For example, the control value can be based upon values of certain markers in comparable samples obtained from a reference cohort. In certain embodiments, the reference cohort is the general population. In certain embodiments, the reference cohort is a select population of human subjects. In certain embodiments, the reference cohort is comprised of individuals who have not previously had any signs or symptoms indicating the presence of atherosclerosis, such as angina pectoris, history of a cardiovascular event such as a myocardial infarction or stroke, evidence of atherosclerosis by diagnostic imaging methods including, but not limited to coronary angiography. In certain embodiments, the reference cohort includes individuals, who if examined by a medical professional would be characterized as free of symptoms of disease (e.g., cardiovascular disease). In another example, the reference cohort may be individuals who are nonsmokers (i.e., individuals who do not smoke cigarettes or related items such as cigars). The threshold values selected may take into account the category into which the test subject falls. Appropriate categories can be selected with no more than routine experimentation by those of ordinary skill in the art. The threshold value is preferably measured using the same units used to measures one or more markers of the present invention.

The threshold value can take a variety of forms. The threshold value can be a single cut-off value, such as a median or mean. The control value can be established based upon comparative groups such as where the risk in one defined group is double the risk in another defined group. The threshold values can be divided equally (or unequally) into groups, such as a low risk group, a medium risk group and a high-risk group, or into quadrants, the lowest quadrant being individuals with the lowest risk the highest quadrant being individuals with the highest risk, and the test subject's risk of having CVD can be based upon which group his or her test value falls. Threshold values for markers in biological samples obtained, such as mean levels, median levels, or "cut-off" levels, are established by assaying a large sample of individuals in the general population or the select population and using a statistical model such as the predictive value method for selecting a positivity criterion or receiver operator characteristic curve that defines optimum specificity (highest true negative rate) and sensitivity (highest true positive rate) as described in Knapp, R. G., and Miller, M. C. (1992). Clinical Epidemiology and Biostatistics. William and Wilkins, Harual Publishing Co. Malvern, Pa., which is specifically incorporated herein by reference. A "cutoff" value can be determined for each risk predictor that is assayed.

Levels of particular markers in a subject's biological sample may be compared to a single threshold value or to a range of threshold values. If the level of the marker in the test subject's biological sample is greater than the threshold value or exceeds or is in the upper range of threshold values, the test subject may, depending on the marker, be at greater risk of developing or having CVD or experiencing a cardiovascular event within the ensuing year, two years, and/or three years than individuals with levels comparable to or below the threshold value or in the lower range of threshold values. In contrast, if levels of the marker in the test subject's biological sample is below the threshold value or is in the lower range of threshold values, the test subject, depending on the marker, be at a lower risk of developing or having CVD or experiencing a cardiovascular event within the ensuing year, two years, and/or three years than individuals whose levels are comparable to or above the threshold value or exceeding or in the upper range of threshold values. The extent of the difference between the test subject's marker levels and threshold value may also useful for characterizing the extent of the risk and thereby determining which individuals would most greatly benefit from certain aggressive therapies. In those cases, where the threshold value ranges are divided into a plurality of groups, such as the threshold value ranges for individuals at high risk, average risk, and low risk, the comparison involves determining into which group the test subject's level of the relevant marker falls.

## Claims

1. A method of determining the risk of a cardiovascular event in a subject comprising:
a) determining the value of first, second, third, fourth, and fifth markers in a biological sample from said subject, wherein said first, second, third, fourth, and fifth markers are different and are selected from the group consisting of: myel-operoxidase (MPO), an F2-isoprostane (F2-IsoP), C-reactive protein (CRP), urinary micro-albumin (UMA); and lipoprotein-associated phospholipase A2 (Lp-PLAZ), and
b) employing said values for said first, second, third, fourth and fifth markers to determine said subject's risk of experiencing a cardiovascular event.

2. The method of Claim 1, wherein said employing said values for said first, second, third, fourth, and fifth markers to determine said subject's risk of experiencing a cardiovascular event comprises comparing said value of said first marker to a first threshold value, comparing said value of said second marker to a second threshold value, comparing said value of said third marker to a third threshold value, comparing said value of said fourth marker to a fourth threshold value, and comparing said value of said fifth marker to a fifth threshold value.

3. The method of Claim 1, wherein said cardiovascular event is a peri-operative cardiovascular event or a cardiovascular event associated with angioplasty.

4. The method of Claim 1, wherein said cardiovascular event is one or more of the following: non-fatal myocardial infarction, stroke, angina pectoris, transient ischemic attacks, congestive heart failure, aortic aneurysm, aortic dissection, and death.

## Patentansprüche

1. Verfahren zur Bestimmung des Risikos eines Herz-Kreislauf-Ereignisses bei einem Patienten, umfassend:
a) die Bestimmung des Wertes eines ersten, zweiten, dritten, vierten und fünften Markers in einer biologischen Probe des Patienten, wobei der erste, zweite, dritte, vierte und fünfte Marker voneinander verschieden sind und aus der aus Myeloperoxidase (MPO), einem F2-Isoprostan (F2-IsoP), C-reaktivem Protein (CRP), Mikroalbumin im Harn (Urinary Micro-Albumin, UMA) und lipoproteinassoziierter Phospholipase A2 (Lp-PLAZ) bestehenden Gruppe ausgewählt sind, und
b) die Verwendung der Werte für den ersten, zweiten, dritten, vierten und fünften Marker zur Bestimmung des Risikos des Patienten, ein Herz-Kreislauf-Ereignis zu erleiden.

2. Verfahren nach Anspruch 1, wobei die Verwendung der Werte für den ersten, zweiten, dritten, vierten und fünften Marker zur Bestimmung des Risikos des Patienten, ein Herz-Kreislauf-Ereignis zu erleiden, den Vergleich des Wertes für den ersten Marker mit einem ersten Schwellenwert, den Vergleich des Wertes für den zweiten Marker mit einem zweiten Schwellenwert, den Vergleich des Wertes für den dritten Marker mit einem dritten Schwellenwert, den Vergleich des Wertes für den vierten Marker mit einem vierten Schwellenwert und den Vergleich des Wertes für den fünften Marker mit einem fünften Schwellenwert umfasst.

3. Verfahren nach Anspruch 1, wobei es sich bei dem Herz-Kreislauf-Ereignis um ein perioperatives Herz-Kreislauf-Ereignis oder ein mit Angioplastie assoziiertes Herz-Kreislauf-Ereignis handelt.

4. Verfahren nach Anspruch 1, wobei es sich bei dem Herz-Kreislauf-Ereignis um eines oder mehrere der Folgenden handelt: nichttödlicher Herzinfarkt, Schlaganfall, Angina pectoris, transiente ischämische Anfälle, dekompensierte Herzinsuffizienz, Aortenaneurysma, Aortendissektion und Tod.

## Revendications

1. Procédé pour déterminer le risque d'un événement cardiovasculaire chez un sujet comprenant :
a) la détermination de la valeur d'un premier, deuxième, troisième, quatrième, et cinquième marqueur dans un échantillon biologique dudit sujet, où ledit premier, deuxième, troisième, quatrième, et cinquième marqueur sont différents et sont sélectionnés dans le groupe consistant en : la myéloperoxydase (MPO), une F2-isoprostane (F2-IsoP), la protéine C-réactive (CRP), la microalbumine urinaire (UMA) ; et la phospholipase A2 associée aux lipoprotéines (Lp-PLAZ), et
b) l'emploi desdites valeurs pour lesdits premier, deuxième, troisième, quatrième, et cinquième marqueurs afin de déterminer ledit risque du sujet de présenter un événement cardiovasculaire.

2. Procédé selon la revendication 1, dans lequel ledit emploi desdites valeurs pour lesdits premier, deuxième, troisième, quatrième, et cinquième marqueurs afin de déterminer ledit risque du sujet de présenter un événement cardiovasculaire comprend la comparaison de ladite valeur dudit premier marqueur à une première valeur seuil, la comparaison de ladite valeur dudit deuxième marqueur à une deuxième valeur seuil, la comparaison de ladite valeur dudit troisième marqueur à une troisième valeur seuil, la comparaison de ladite valeur dudit quatrième marqueur à une quatrième valeur seuil, et la comparaison de ladite valeur dudit cinquième marqueur à une cinquième valeur seuil.

3. Procédé selon la revendication 1, dans lequel ledit événement cardiovasculaire est un événement cardiovasculaire peropératoire ou un événement cardiovasculaire associé à une angioplastie.

4. Procédé selon la revendication 1, dans lequel ledit événement cardiovasculaire est un ou plusieurs parmi les suivants : un infarctus du myocarde non fatal, un accident vasculaire cérébral, l'angine de poitrine, des accidents ischémiques transitoires, l'insuffisance cardiaque congestive, un anévrisme aortique, une dissection aortique, et le décès.
